# EUROPEAN PATENT SPECIFICATION

(11) **EP 1 488 766 B1**
(45) Date of publication and mention of the grant of the patent: **15.11.2006**
(21) Application number: 04253623.5
(22) Date of filing: 17.06.2004
(51) Int. Cl.: A61F 5/56, A47G 9/10

(54) **Amelioration of snoring**
Linderung des Schnarchens
Allégement du ronflement

(30) Priority: 18.06.2003 GB 0314128
(43) Date of publication of application: 22.12.2004
(73) Proprietor: Screen, Harold, Stourport, Worcestershire DY13 8UG (GB)
(72) Inventor: Screen, Harold, Stourport, Worcestershire DY13 8UG (GB)
(74) Representative: Carpenter, David

(56) References cited:
- DE-A- 2 931 948
- DE-A- 19 520 074
- DE-C- 19 602 825
- GB-A- 2 303 544
- US-A- 5 123 132

## Description

This invention relates to use of a cork sheet for the amelioration of snoring.

Snoring is a coarse sound made by vibrations of the soft palate and other tissues of the upper airway, and is caused by a partial obstruction of the upper airway. Muscle tone normally acts to help keep the airways open. However, during sleep, muscle tone is generally decreased, leading to a narrowing of the airways, and thus an increased likelihood of snoring.

Snoring is a relatively common condition; the British Snoring & Sleep Apnoea Association estimate that 41.5% of the UK adult population snore. Snoring often results in sleep disturbances for not only the sufferers, but also for their respective spouses whose sleep may be adversely affected by the snoring noises or the repeated waking of the sufferer.

There have been many treatments devised for the amelioration of snoring, ranging from sprays to lubricate the throat, devices which act to mechanically keep the airways open, to surgical procedures.

Document US-A-5 123 132 discloses the use of a pillow made of polyurethane foam for the amelioration of snoring.

It is an object of the present invention to ameliorate snoring in a simple and inexpensive manner.

In accordance with the present invention, a cork sheet for the amelioration of snoring is placed beneath the user's head, such that at least part of the cork sheet lies either directly or indirectly beneath at least part of the user's head.

Preferably, the cork sheet is rectangular in profile, and desirably the sheet has a thickness of at least 1mm, and preferably no more than 10mm.

Cork is derived from naturally occurring sources, and is known for its antivibration, and sound-deadening properties. It is a non-toxic material, and is obtainable from environmentally friendly sources. The applicant has found, through experiment, that the presence of a cork sheet beneath a user's head during sleep is of benefit for the amelioration of snoring. It is currently believed that the cork sheet acts to ameliorate snoring by mechanically dampening the vibrations produced during snoring.

Preferably, the cork sheet is encased within a bag, and advantageously the bag is closed. Conveniently the bag is of cotton material, and desirably, the cotton material is at least partly of a light colour. Preferably, the bag is formed from a material that is impermeable to water, and desirably said bag is formed from plastics material. Advantageously, the bag is sealed to prevent water ingress, and conveniently the bag is sealed with glue.

Advantageously, where the bag is air permeable, the bag also contains material which is thought to encourage a good night's sleep, for example having relaxing or soporific properties, and preferably such material comprises dried hops, dried lavender, or a mixture of dried hops and dried lavender. Alternatively, the bag contains material having decongestant properties. Conveniently, said material has both relaxing or soporific, and decongestant properties. Preferably, the material is contained within a sachet, and advantageously the sachet is permeable to the air. Preferably, in use, said material is disposed beneath the cork sheet.

Preferably, at least part of the cork sheet is treated with a dye, such that in use, when the cork sheet is positioned within the bag, if the cork sheet becomes damp or wet, the dye stains the cotton sheet and is then visible to a user. Advantageously, the dye is vegetable-based.

Preferably, the cork sheet is at least partly covered in a cushioning material, and advantageously said cushioning material is bubble wrap. Preferably, the cork sheet is affixed to a sheet of relatively rigid material, and desirably the cork sheet is affixed to the sheet of relatively rigid material by glue. Conveniently, the relatively rigid material is cardboard.

Advantageously, the device is placed between the user's pillow and a bed.

The present invention will now be described, by way of example only, with reference to the accompanying drawings, in which:
Figure 1 is a schematic cross-sectional side view of a preferred embodiment of a snoring amelioration device
Figure 2 is a reduced scale perspective view of the cork sheet of Figure 1, and
Figure 3 is a perspective view of the cork sheet of Figures 1 and 2 in place within a bag.

Referring to the drawings, one embodiment of a snoring amelioration device 10 comprises a cork sheet 11, and a sachet 12 containing material such as a mixture of dried hops and lavender 13, both contained within a bag 14.

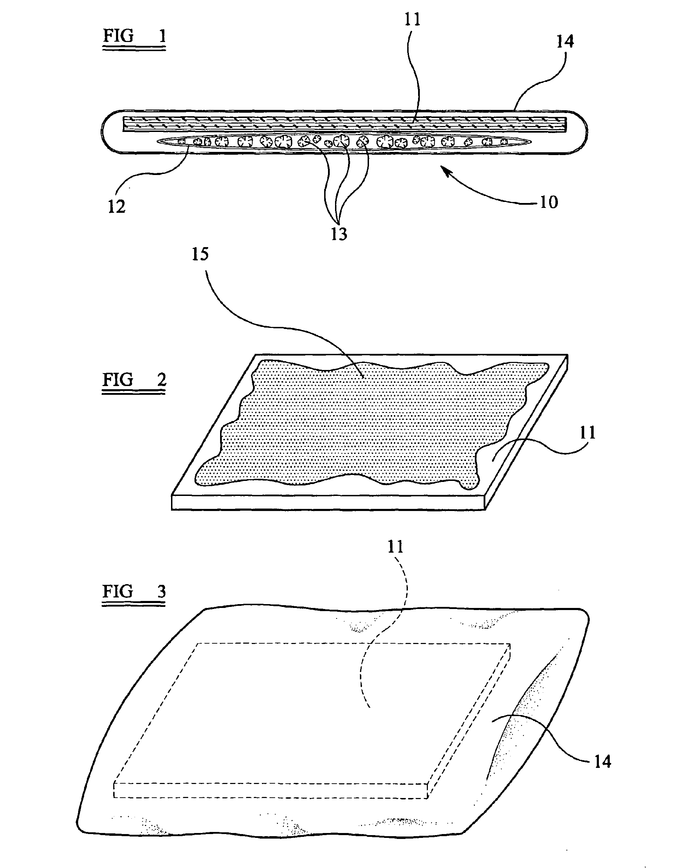

Dried hops and lavender are known as traditional sleep aids, as they are thought to have relaxing or soporific effects, and have a pleasant aroma. It will be understood that such materials are not essential to the invention, and that the use of the cork sheet itself is key to the amelioration of snoring. It will also be understood that other materials may be used within the sachet, either instead, or in addition to the hops and lavender mixture. For example, a substance impregnated with a decongestant, or aromatherapy oil may be used. It is desirable that the sachet 12 containing the material such as dried hops and lavender, and the bag 14, are permeable to the air, in order for the aroma of the material contained therein to reach the user. For example, a paper sachet may be used, although other materials such as muslin or cotton would be equally acceptable. In use, the sachet 12 containing the material such as dried hops and lavender 12 is disposed beneath the cork sheet 11 within the bag 14, as can be seen from Figure 1. It will be appreciated that if material such as dried hops and lavender is used, it need not be placed within a sachet, and may be placed directly within the bag. Furthermore, it will be appreciated that if such materials are not used, the bag need not be permeable to the air.

Although the cork sheet as described and shown in Figures 2 and 3 is of a substantially rectangular shape, the shape of the sheet is not critical. In fact many other shapes of sheet could be used, such as oval or round sheets, as long as the sheet is of a suitable size and shape such that, in use, a user's head lies, either directly or indirectly, substantially above the sheet.

The applicant has found the thickness of cork sheet optimal for the amelioration of snoring to be in the order of 3mm. However, in practice, a range of thicknesses would be suitable. In order to have any noticeable impact on the sufferer's snoring, the sheet should be of at least 1mm thickness. The sheet should preferably be of no more than 10mm thickness. In a preferred embodiment, the cork sheet is glued to a sheet of cardboard (not shown) in order to minimise the risk of the cork sheet being damaged. It will be appreciated that means other than glue, for example mechanical fixing means, may be used to secure the cork sheet to the cardboard. Furthermore, it will be appreciated that alternative materials to cardboard may be used, as long as they are relatively rigid materials which will act to prevent damage to the cork sheet. For example, a sheet of plastics material may be used. The applicant has found that the efficacy of the cork sheet for the amelioration of snoring is reduced if the sheet is allowed to become wet. In the illustrated embodiment, at least part of the sheet is treated with a vegetable dye 15, as seen in Figure 2. If the sheet 11 does become wet, the dye will run, and will soak into any material of the bag 14 that is in contact with the sheet. It will therefore be evident if the cork sheet has been in contact with liquid, and a replacement sheet may then be obtained. It can be seen that it is beneficial that a relatively dark dye 15 is used, and a bag 14 which is at least partly relatively light in colour, is used. If a sachet of material such as dried hops and lavender is also present within the bag 14, it will be appreciated that the vegetable dye need only be present in areas where the dye is in contact with the bag, i.e. on the upper surface of the cork sheet.
The cotton bag 14 as shown in Figures 1 and 3, is preferably closed so that the cork sheet and sachet are contained therein. However, it will be understood that it is not necessary to close the bag. Alternatively, the bag may be releasably closed, by means such as a zip or Velcro^{®}, which may be of benefit if, for example, the user desires a change of material contained within the sachet, or a change of sachet itself. In a preferred embodiment, the bag is made from cotton. However, various materials may be employed. If a sachet containing material such as dried hops and lavender is not present within the bag, then it is not necessary to use an air-permeable material for the bag, and a waterproof material may be used in order to protect the cork sheet from becoming wet, in which case the vegetable dye will not be necessary. In addition, it is important to note that where such a sachet is not present, it is not essential to have a bag at all.

In an alternative embodiment (which has not been illustrated), the cork sheet is kept dry by being sealed in a waterproof bag, for example a plastic bag. The plastic bag is preferably sealed by way of a glue strip, although it will be appreciated that other ways of sealing the bag may be used, such as by way of a heat sealing method. It will be evident that, should a waterproof bag be used, dried hops and lavender need not be used, since the aroma will not reach the user. In such an alternative embodiment, treatment of the cork sheet with vegetable dye is not required, as mentioned above.

For enhanced user comfort, the cork sheet may be covered in bubble wrap (not shown). It will be appreciated that bubble wrap may be used to cover both the embodiment in which the cork sheet is glued to a sheet of cardboard, and the embodiment in which no cardboard sheet is used, and that in this respect, references a the cork sheet include references to a cork sheet which has been glued to a cardboard sheet. It will further be appreciated that alternative cushioning materials may be used to cover the cork sheet, such as foam. It will also be appreciated that the cork sheet need not be entirely covered in the cushioning material, and that only the part which is likely to be disposed underneath a user's head may be covered in the cushioning material. In this embodiment, the cork sheet covered in cushioning material is contained within the bag 14.

In use, the device 10 is placed either directly or indirectly beneath the user's head. A preferred use involves the device being placed on a bed, such that when the user of the device is in the bed in their usual sleeping position, the user's head will substantially overlie at least part of the device. The user may place his head directly onto the device, or the device may be placed beneath the user's pillow. Tests indicate that where a user supports his head with more than one pillow then the device should be positioned immediately beneath the upper pillow and that the efficacy of the device may be reduced if it is disposed beneath more than one pillow. It is important that the device is disposed such that at least part of the cork sheet 11 lies either directly or indirectly beneath at least part of the user's head during sleep.

## Claims

1. Use of a cork sheet (11) for the amelioration of snoring, wherein the cork sheet (11) is placed beneath a user's head, such that at least part of the cork sheet lies either directly or indirectly beneath at least part of the user's head.

2. Use of a cork sheet (11) as claimed in Claim 1, wherein the cork sheet has a thickness of at least 1mm.

3. Use of a cork sheet (11) as claimed in either one of the preceding claims, wherein the cork sheet has a thickness of no more than 10mm.

4. Use of a cork sheet (11) as claimed in any one of the preceding claims, wherein the cork sheet is encased within a bag (14).

5. Use of a cork sheet (11) as claimed in Claim 4, wherein the bag is formed from cotton material.

6. Use of a cork sheet (11) as claimed in either one of Claims 4 or 5, wherein the bag contains material which is thought to encourage a good night's sleep, for example having relaxing or soporific properties.

7. Use of a cork sheet (11) as claimed in any one of Claims 4 to 6, wherein at least part of the cork sheet is treated with a dye (15), such that when the cork sheet is positioned within the bag, if the cork sheet becomes damp or wet, the dye stains the bag and is then visible to a user.

8. Use of a cork sheet (11) as claimed in Claim 4, wherein the bag is formed from a material which is impermeable to water.

9. Use of a cork sheet (11) as claimed in any one of the preceding claims, wherein the cork sheet is at least partly covered in a cushioning material.

10. Use of a cork sheet (11) as claimed in any one of the preceding claims, wherein the cork sheet is affixed to a sheet of relatively rigid material.

11. Use of a cork sheet (11) as claimed in Claim 10, wherein the cork sheet is affixed to the sheet of relatively rigid material by glue.

12. Use of a cork sheet (11) as claimed in the preceding claim, wherein the cork sheet (11) is placed between the user's pillow and a bed.

## Patentansprüche

1. Verwendung einer Korkplatte (11) zur Linderung des Schnarchens, wobei die Korkplatte (11) unterhalb des Kopfes eines Benutzers angeordnet wird, so dass mindestens ein Teil der Korkplatte entweder direkt oder indirekt unterhalb mindestens eines Teils des Kopfes des Benutzers liegt.

2. Verwendung einer Korkplatte (11) nach Anspruch 1, wobei die Korkplatte eine Dicke von mindestens 1 mm aufweist.

3. Verwendung einer Korkplatte (11) nach einem der beiden der vorhergehenden Ansprüche, wobei die Korkplatte eine Dicke von nicht mehr als 10 mm aufweist.

4. Verwendung einer Korkplatte (11) nach einem der vorhergehenden Ansprüche, wobei die Korkplatte innerhalb eines Beutels (14) eingeschlossen ist.

5. Verwendung einer Korkplatte (11) nach Anspruch 4, wobei der Beutel aus Baumwollmaterial hergestellt wird.

6. Verwendung einer Korkplatte (11) nach einem der beiden der Ansprüche 4 oder 5, wobei der Beutel Material enthält, von dem man glaubt, dass es einen guten Schlaf in der Nacht unterstützt, indem es beispielsweise entspannende Eigenschaften aufweist oder einschläfernd wirkt.

7. Verwendung einer Korkplatte (11) nach einem der Ansprüche 4 bis 6, wobei mindestens ein Teil der Korkplatte mit einem Farbstoff (15) behandelt ist, so dass, wenn die Korkplatte innerhalb des Beutels positioniert ist, wenn die Korkplatte feucht oder nass wird, der Farbstoff den Beutel verfärbt und dann für einen Benutzer sichtbar wird.

8. Verwendung einer Korkplatte (11) nach Anspruch 4, wobei der Beutel aus einem Material gebildet wird, das wasserdicht ist.

9. Verwendung einer Korkplatte (11) nach einem der vorhergehenden Ansprüche, wobei die Korkplatte mindestens teilweise in ein Polstermaterial eingehüllt ist.

10. Verwendung einer Korkplatte (11) nach einem der vorhergehenden Ansprüche, wobei die Korkplatte an einer Platte aus einem relativ steifen Material befestigt ist.

11. Verwendung einer Korkplatte (11) nach Anspruch 10, wobei die Korkplatte an der Platte aus dem relativ steifen Material mittels Klebstoff befestigt ist.

12. Verwendung einer Korkplatte (11) nach einem der vorhergehenden Ansprüche, wobei die Korkplatte (11) zwischen dem Kissen des Benutzers und einem Bett angeordnet wird.

## Revendications

1. Utilisation d'une plaque de liège (11) pour réduire le ronflement, la plaque de liège (11) étant placée au-dessous de la tête d'un utilisateur, de sorte qu'au moins une partie de la plaque de liège se situe directement ou indirectement au-dessous d'au moins une partie de la tête de l'utilisateur.

2. Utilisation d'une plaque de liège (11) selon la revendication 1, dans laquelle la plaque de liège a une épaisseur d'au moins 1 mm.

3. Utilisation d'une plaque de liège (11) selon l'une des revendications précédentes, dans laquelle la plaque de liège a une épaisseur non supérieure à 10 mm.

4. Utilisation d'une plaque de liège (11) selon l'une quelconque des revendications précédentes, dans laquelle la plaque de liège est renfermée dans un sac (14).

5. Utilisation d'une plaque de liège (11) selon la revendication 4, dans laquelle le sac est composé d'un matériau de coton.

6. Utilisation d'une plaque de liège (11) selon l'une des revendications 4 ou 5, dans laquelle le sac contient un matériau destiné à assurer un bon sommeil durant la nuit, présentant par exemple des propriétés relaxantes ou soporifiques.

7. Utilisation d'une plaque de liège (11) selon l'une quelconque des revendications 4 à 6, dans laquelle au moins une partie de la plaque de liège est traitée par un colorant (15), de sorte que lorsque la plaque de liège et positionnée dans le sac, la plaque de liège étant humide ou mouillée, le colorant colore le sac, ceci étant visible par un utilisateur.

8. Utilisation d'une plaque de liège (11) selon la revendication 4, dans laquelle le sac est composé d'un matériau imperméable à l'eau.

9. Utilisation d'une plaque de liège (11) selon l'une quelconque des revendications précédentes, dans laquelle la plaque de liège est au moins partiellement recouverte d'un matériau de protection.

10. Utilisation d'une plaque de liège (11) selon l'une quelconque des revendications précédentes, dans laquelle la plaque de liège est fixée sur une plaque de matériau relativement rigide.

11. Utilisation d'une plaque de liège (11) selon la revendication 10, dans laquelle la plaque de liège est fixée sur la plaque de matériau relativement rigide par une colle.

12. Utilisation d'une plaque de liège (11) selon l'une quelconque des revendications précédentes, dans laquelle la plaque de liège est placée entre le coussin de l'utilisateur et un lit.
